# EUROPÄISCHE PATENTANMELDUNG

(11) **EP 4 850 648 A1**
(43) Veröffentlichungstag der Anmeldung: **09.12.2026**
(21) Anmeldenummer: 26405006.3
(22) Anmeldetag: 21.05.2026
(51) Int. Cl.: A61F 13/26

(54) **SPIRALGLEITER ZUM EINFÜHREN VON TAMPONS**

(30) Priorität: 06.06.2025 CH 6182025
(71) Anmelder: TAMPiNO GmbH Ursula Salathé, 4148 Pfeffingen (CH)
(72) Erfinder: Salathé, Ursula, 4148 Pfeffingen (CH)

(57) **Zusammenfassung**

Die Erfindung betrifft einen wiederverwendbaren Spiralgleiter (1) zur Einführung handelsüblicher Tampons ohne Applikator. Der Spiralgleiter weist einen spiralförmigen Körper mit einem durchgehenden Schlitz (2) auf, der eine elastische Ausweitung in radialer Richtung ermöglicht, sowie einen erweiterten Griffbereich (3) am unteren Endbereich und eine innenliegende Lasche (4), die Tampons unterschiedlicher Grössen formschlüssig oder klemmend fixiert. Die Länge des spiralförmigen Körpers gewährleistet eine ausreichend tiefe Platzierung des Tampons im Vaginalkanal, während die über das obere Ende des Spiralgleiters hinausragende Tamponspitze eine geführte und komfortable Einführung ermöglicht. Der Spiralgleiter ist aus einem flexiblen, biokompatiblen und hygienisch reinigbaren Material, insbesondere einem Elastomer, Silikon, TPE oder TPU gefertigt und für den wiederverwendbaren Einsatz konzipiert. Die Erfindung trägt zur Reduzierung von Einwegmüll bei und ermöglicht eine einfache hygienische Reinigung. Der Spiralgleiter wird zusammen mit einem Schiebeelement verwendet; das Schiebeelement und ein Deckel dienen ausschliesslich der Handhabung und Hygiene, wodurch eine handliche und hygienische Gesamteinheit entsteht, und sind nicht Gegenstand der Schutzansprüche.

## Beschreibung

### TECHNISCHES GEBIET DER ERFINDUNG

Die Erfindung betrifft eine Vorrichtung zur hygienischen Einführung von Tampons, insbesondere einen wiederverwendbaren Spiralgleiter (Applikator), der handelsübliche Tampons verschiedener Grössen ohne integrierten Applikator aufnehmen kann und eine einfache, hygienische sowie präzise Einführung ermöglicht.

### STAND DER TECHNIK

Bekannte Applikatoren für Tampons bestehen typischerweise aus Kunststoff oder Pappe und sind ausschliesslich für den einmaligen Gebrauch vorgesehen. Dies führt zu erheblichen Mengen an Verpackungs- und Produktabfall.

Bestehende Applikatorlösungen weisen wesentliche Nachteile auf: Sie sind überwiegend nicht kompatibel mit handelsüblichen Tampons verschiedener Grössen und erfordern herstellerspezifische Produkte. Darüber hinaus ist die Gesamtlänge herkömmlicher Applikatoren häufig unzureichend, um eine korrekte Platzierung des Tampons zu gewährleisten. Sitzt der Tampon im vorderen Drittel der Vagina, ist er für die Anwenderin spürbar und verursacht Unbehagen. Eine korrekte Platzierung erfordert daher einen Applikator mit ausreichender Länge, der den Tampon tief genug in den Vaginalkanal einzuführen vermag.

Es besteht daher ein Bedarf nach einer wiederverwendbaren, hygienisch einwandfreien und anwenderfreundlichen Lösung, die eine kontrollierte Einführung von Tampons ermöglicht.

### AUFGABE DER ERFINDUNG

Aufgabe der Erfindung ist es, ein anwenderfreundliches, wiederverwendbares und hygienisch nutzbares Hilfsmittel bereitzustellen, welches eine kontrollierte und angenehme Einführung handelsüblicher Tampons unterschiedlicher Grössen ermöglicht.

Die Vorrichtung unterstützt insbesondere eine schmerzreduzierte und stressfreie Platzierung des Tampons bei eingeschränkter Beweglichkeit, bei krampfbedingten Beschwerden während der Menstruation sowie bei vaginaler Trockenheit.

Die ergonomische Gestaltung des Spiralgleiters ermöglicht das hygienische Einführen eines Tampons in aufrechter Körperhaltung, ohne dass eine gebückte oder unnatürliche Haltung erforderlich ist.

Die Vorrichtung ist für die Verwendung durch Erstanwenderinnen von Tampons sowie für Personen geeignet, die bisher ausschliesslich Binden verwendet haben, und erleichtert eine hygienische sowie präzise Einführung. Darüber hinaus fördert sie die Nutzung ökologischer Baumwolltampons und trägt zur Reduzierung von Einwegmüll bei.

Ein weiterer Vorteil besteht darin, dass auch Tampons mit rauer Oberfläche, insbesondere solche aus ungebleichter Baumwolle oder ohne Kunststoffbeschichtung, angenehm und schmerzfrei eingeführt werden können. Baumwolltampons ohne Kunststoffbeschichtung weisen gegenüber herkömmlichen kunststoffbeschichteten Tampons ökologische sowie gesundheitliche Vorteile auf, sind in der Praxis jedoch aufgrund ihrer rauen Oberfläche schwerer einzuführen. Der Spiralgleiter ermöglicht hier ein komfortables und hygienisches Einführen, wodurch der Gebrauch umwelt- und gesundheitsfreundlicher Tampons gefördert wird.

Darüber hinaus eignet sich der Spiralgleiter für Anwenderinnen mit langen Fingernägeln, da er das Einführen des Tampons inklusive Rückholschnur ohne direkten Kontakt zwischen Hand und Vaginalbereich ermöglicht, was das Risiko von Verunreinigungen reduziert und eine verbesserte hygienische Handhabung gewährleistet.

### LÖSUNG DER AUFGABE

Die Aufgabe wird durch einen Spiralgleiter gemäss Anspruch 1 gelöst. Vorteilhafte Weiterbildungen sind Gegenstand der abhängigen Ansprüche.

Der erfindungsgemässe Spiralgleiter (1) umfasst einen spiralförmig ausgebildeten Körper, der entlang seiner Längsachse einen durchgehenden Schlitz (2) aufweist, welcher ein elastisches Ausweiten der Spirale in radialer Richtung ermöglicht, sodass Tampons unterschiedlicher Durchmesser in den Innenbereich der Spirale eingebracht werden können; einen erweiterten Griffbereich (3) am unteren Endbereich des Spiralkörpers, welcher das Greifen und kontrollierte Führen ermöglicht; sowie eine innenliegende Lasche (4), die sich in den Innenraum der Spirale erstreckt und dazu ausgebildet ist, Tampons unterschiedlicher Durchmesser formschlüssig oder klemmend zu fixieren, wobei die Lasche wahlweise im Innenraum zur formschlüssigen oder klemmenden Fixierung kleinerer Tampons oder aussenliegend zur Fixierung grösserer Tampons verwendet werden kann.

### KURZE BESCHREIBUNG DER ZEICHNUNGEN

Die Erfindung wird nachfolgend anhand von Ausführungsbeispielen und der beigefügten Zeichnungen näher erläutert. Es zeigen:
Fig. 1: eine Frontalansicht des Spiralgleiters (1) mit sichtbarem Schlitz (2) und Griffbereich (3);
Fig. 2: eine Aufsicht des Spiralgleiters (1) von oben mit erkennbarer spiralförmiger Anordnung, dem durchgehenden Schlitz (2) sowie der innenliegenden Lasche (4);
Fig. 3: eine Untersicht des Spiralgleiters (1) von unten mit sichtbarer ringförmiger Struktur, Schlitzposition (2) und Lasche (4);
Fig. 4: eine perspektivische Ansicht des Spiralgleiters (1) mit Bezugsziffern 1 - 4, insbesondere mit in den Innenraum ragender Lasche (4) in der Position für kleinere Tampons;
Fig. 5: eine perspektivische Gesamtansicht des Spiralgleiters (1) mit sämtlichen Bezugsziffern 1 - 4, welche die räumliche Anordnung von Spiralkörper (1), Schlitz (2), Griffbereich (3) und Lasche (4) in ihrer funktionalen Gesamtheit zeigt;
Fig. 6: eine Draufsicht auf den vollständig abgewickelten Spiralgleiter (1) mit über ihre gesamte Länge sichtbarer Lasche (4) und Griffbereich (3).

### AUSFÜHRLICHE BESCHREIBUNG DER ERFINDUNG

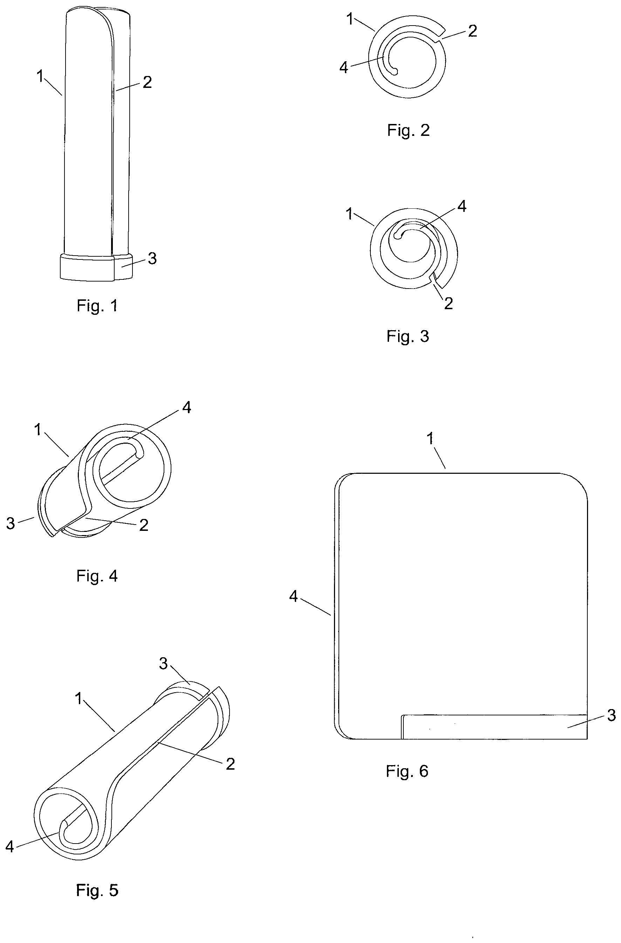

Anhand der Fig. 1 bis Fig. 6 wird nachfolgend ein bevorzugtes Ausführungsbeispiel des erfindungsgemässen Spiralgleiters (1) beschrieben.

Der Spiralgleiter (1) weist einen spiralförmig ausgebildeten Körper auf, der entlang seiner Längsachse einen durchgehenden Schlitz (2) besitzt (vgl. Fig. 1, Fig. 2, Fig. 3). Dieser Schlitz ermöglicht ein kontrolliertes elastisches Ausweiten der Spirale in radialer Richtung, wodurch Tampons unterschiedlicher Durchmesser in den Innenbereich der Spirale eingebracht werden können.

Am unteren Endbereich des Spiralkörpers ist ein erweiterter Griffbereich (3) angeordnet (Fig. 1, Fig. 4, Fig. 5, Fig. 6), der eine ergonomische und intuitive Handhabung bei der Anwendung sicherstellt.

Die Länge des spiralförmigen Körpers ist ferner derart bemessen, dass die Spitze eines korrekt eingeschobenen Tampons inklusive Rückholschnur aus dem oberen Ende des Spiralgleiters herausragt. Diese Gestaltung erfüllt zwei Funktionen: Einerseits zeigt die herausragende Tamponspitze der Anwenderin an, dass der Tampon vollständig und korrekt im Spiralgleiter fixiert ist. Andererseits ermöglicht die vorangehende Tamponspitze eine geführte und komfortable Einführung des Spiralgleiters in den Vaginalkanal, da sie den Weg vorgibt und eine präzise Platzierung des Tampons in der richtigen Tiefe unterstützt.

Eine innenliegende Lasche (4) erstreckt sich in den Innenraum der Spirale (Fig. 2, Fig. 3, Fig. 4, Fig. 5, Fig. 6). Die Lasche ist so ausgebildet, dass sie wahlweise im Innenraum verbleibt und dort kleine Tampons formschlüssig oder klemmend fixiert, oder nach aussen umgelegt wird, sodass sie aussen am Spiralgleiter anliegt und durch die elastische Dehnung des Körpers grössere Tampons sicher fixiert.

Fig. 5 zeigt den Spiralgleiter (1) in einer perspektivischen Gesamtansicht mit sämtlichen Bezugsziffern 1 - 4 und veranschaulicht die räumliche Anordnung von Spiralkörper (1), Schlitz (2), Griffbereich (3) und Lasche (4) in ihrer funktionalen Gesamtheit.

Fig. 6 zeigt den Spiralgleiter (1) in vollständig abgewickeltem Zustand. In dieser Darstellung ist die Lasche (4) über ihre gesamte Länge erkennbar.

Der Spiralgleiter ist aus einem flexiblen, biokompatiblen und hygienisch reinigbaren Material gefertigt, insbesondere aus einem Elastomer, Silikon, thermoplastischen Elastomer (TPE), thermoplastischem Polyurethan (TPU) oder einem gleichwertigen biokompatiblen Werkstoff. Er ist für den wiederverwendbaren Einsatz konzipiert und kann durch Waschen oder Auskochen desinfiziert werden.

Zur Anwendung wird ein Tampon (ohne eigenen Applikator) von unten in den Spiralgleiter (1) eingeschoben. Der durchgehende Schlitz (2) entlang der Längsachse des spiralförmigen Körpers ermöglicht dabei eine elastische Ausweitung der Spirale in radialer Richtung, sodass sich der Spiralkörper dem jeweiligen Tampondurchmesser anpasst und handelsübliche Tampons unterschiedlicher Grössen aufgenommen werden können. Die Lasche (4) ist so ausgestaltet, dass sie den eingeschobenen Tampon unabhängig von dessen Durchmesser formschlüssig oder klemmend im Innern der Spirale fixiert. Der Spiralgleiter wird sodann manuell eingeführt. Durch gezielten Druck mit dem Schiebeelement wird der Tampon kontrolliert aus dem Spiralgleiter in den Vaginalkanal geschoben. Der Spiralgleiter wird anschliessend entnommen und gereinigt.

Der Spiralgleiter (1) wird zur Anwendung zusammen mit einem Schiebeelement verwendet, welches dazu dient, den in den Spiralgleiter eingebrachten Tampon durch axialen Druck kontrolliert aus dem Spiralgleiter in den Vaginalkanal zu schieben. Das Schiebeelement ist somit Bestandteil der funktionalen Gesamteinheit. Ferner ist ein Deckel vorgesehen, welcher ausschliesslich der Handhabung und Hygiene dient und zusammen mit dem Schiebeelement eine handliche und hygienische Gesamteinheit bildet. Das Schiebeelement und der Deckel sind nicht Gegenstand der vorliegenden Patentansprüche.

## Patentansprüche

1. Spiralgleiter (1) zur Aufnahme und kontrollierten Einführung von Tampons unterschiedlicher Grössen, umfassend einen spiralförmig ausgebildeten Körper, der entlang seiner Längsachse einen durchgehenden Schlitz (2) aufweist, wobei der Körper eine elastische Ausweitung in radialer Richtung ermöglicht, **dadurch gekennzeichnet, dass** der untere Endbereich einen erweiterten Griffbereich (3) aufweist sowie eine innenliegende Lasche (4), die sich in den Innenraum der Spirale erstreckt und dazu ausgebildet ist, Tampons unterschiedlicher Durchmesser formschlüssig oder klemmend zu fixieren.

2. Spiralgleiter nach Anspruch 1, **dadurch gekennzeichnet, dass** die Lasche (4) wahlweise im Innenraum zur formschlüssigen oder klemmenden Fixierung kleinerer Tampons eingesetzt oder aussenliegend zur Fixierung grösserer Tampons verwendet werden kann.

3. Spiralgleiter nach einem der vorhergehenden Ansprüche, **dadurch gekennzeichnet, dass** der spiralförmige Körper aus einem flexiblen, biokompatiblen und hygienisch reinigbaren Material gefertigt ist, insbesondere aus einem Elastomer, Silikon, thermoplastischen Elastomer (TPE), thermoplastischem Polyurethan (TPU) oder einem gleichwertigen biokompatiblen Werkstoff, welches eine Wiederverwendbarkeit des Spiralgleiters erlaubt.

4. Spiralgleiter nach einem der vorhergehenden Ansprüche, **dadurch gekennzeichnet, dass** der Schlitz (2) über die gesamte Länge des spiralförmigen Körpers verläuft, um ein kontrolliertes elastisches Ausweiten der Spirale beim Einführen des Tampons zu ermöglichen.

5. Spiralgleiter nach einem der vorhergehenden Ansprüche, **dadurch gekennzeichnet, dass** die Länge des spiralförmigen Körpers derart bemessen ist, dass die Spitze eines korrekt eingesetzten Tampons über das obere Ende des Spiralgleiters hinausragt.

6. Spiralgleiter nach einem der vorhergehenden Ansprüche, **dadurch gekennzeichnet, dass** der Griffbereich (3) am unteren Endbereich des spiralförmigen Körpers eine gegenüber dem Spiralkörper verbreiterte Form aufweist, die ein sicheres Greifen und eine kontrollierte axiale Führung bei der Anwendung ermöglicht.
